# EUROPEAN PATENT APPLICATION

(11) **EP 4 215 531 A1**
(43) Date of publication of application: **26.07.2023**
(21) Application number: 21868728.3
(22) Date of filing: 17.09.2021
(51) Int. Cl.: C07D 455/03, A61K 31/4375, A61P 1/16

(54) **COMPOUND FOR PREVENTING AND TREATING LIVER DISEASES AND PHARMACEUTICAL USE THEREOF**

(30) Priority: 18.09.2020 CN 202010986530
(71) Applicant: Chengdu Biobel Company Limited, Chengdu, Sichuan 610093 (CN)
(72) Inventor: GUANG, Bing, Chengdu, Sichuan 610093 (CN); YANG, Tai, Chengdu, Sichuan 610093 (CN); DONG, Renhan, Chengdu, Sichuan 610093 (CN); ZHAN, Wei, Chengdu, Sichuan 610093 (CN); LIU, Jin, Chengdu, Sichuan 610093 (CN); QIN, Chuanjun, Chengdu, Sichuan 610093 (CN); XIE, Jian, Chengdu, Sichuan 610093 (CN); HUANG, Sheng, Chengdu, Sichuan 610093 (CN); PENG, Xiangyang, Chengdu, Sichuan 610093 (CN); LAI, Yongxin, Chengdu, Sichuan 610093 (CN); XU, Qing, Chengdu, Sichuan 610093 (CN); PENG, Jian, Chengdu, Sichuan 610093 (CN); ZENG, Yisheng, Chengdu, Sichuan 610093 (CN); HU, Xiaojun, Chengdu, Sichuan 610093 (CN)
(74) Representative: Altmann Stößel Dick Patentanwälte PartG mbB
(86) International application number: PCT/CN2021/119131
(87) International publication number: WO 2022/057906

(57) **Abstract**

Disclosed is a compound for preventing and treating liver diseases and the pharmaceutical use thereof, which belong to the field of medical technology. Specifically disclosed is a compound as represented by formula 1-1, or a pharmaceutically acceptable salt, a stereoisomer, or a solvate thereof, which can not only significantly reduce ALT, AST and ALP, but which also has a significant improvement effect on hepatic pathological injuries (e.g. hepatocyte steatosis). The therapeutic effect of the compound on fatty liver, hepatic fibrosis and liver cirrhosis is better than that of the reported compound, and the compound has wide application prospects in the preparation of a drug for preventing and/or treating liver diseases such as fatty liver, hepatic fibrosis and liver cirrhosis.

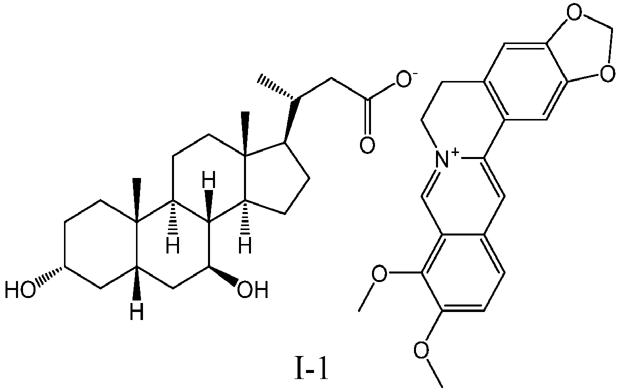

## Description

### Technical field

The present invention belongs to the technical field of medicine, and in particular relates to a compound for preventing and treating liver diseases and the pharmaceutical use thereof.

### Background technology

Fatty liver disease (FLD) is a common liver disease, which refers to the hepatic lipid accumulation caused by various reasons, and when the fat content exceeds 5% of the liver weight (wet weight) or 30% of the liver parenchyma histologically, that is called fatty liver disease. FLD can be subdivided into alcoholic fatty liver disease (AFLD) or nonalcoholic fatty liver disease (NAFLD). The latter further includes obesity fatty liver, dystrophic fatty liver, drug-induced fatty liver, acute fatty liver of pregnancy (AFLP), diabetic fatty liver (DFL), and so on. In terms of the courses of disease, FLD can be divided into three stages: stage I is simple fatty liver; stage II is fatty hepatitis, about 10% of which is transformed into stage III; and stage III is steatohepatic fibrosis and hepatic cirrhosis. FLD is the second largest liver disease after hepatitis in China, and the incidence rate is still rising steadily; FLD patients are more than hepatitis patients in Europe and America, where FLD is also one of the top ten common causes of death. In short, FLD has become a serious problem threatening human life and health, but the drugs for preventing and treating FLD can not meet the clinical needs.

It has been reported that cholic acid derivatives have certain effects in the treatment of FLD. The norursodeoxycholic acid (norUDCA) has been reported to have the effect of preventing and treating NAFLD (Journal of Hepatology, 2010, 52: S304), and have a better therapeutic effect on cholestatic cirrhosis (Journal of Hepatology, 2017,67:549), and also have a better protective effect in the model of liver fibrosis induced by thioacetamide. HTD1801 is a candidate drug molecule for AFLD and primary sclerosing cholangitis (PSC), having a structural formula shown below, which is composed of ursodeoxycholic acid and berberine. However, the therapeutic effect of these compounds on FLD needs to be further improved.

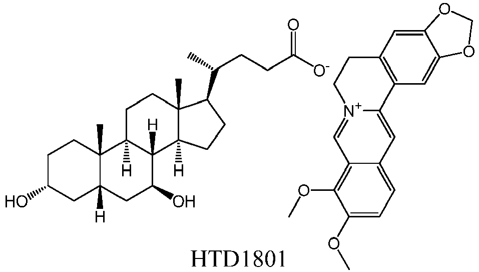

In brief, there is an urgent need to develop safe and effective new drugs for the treatment of FLD.

### Content of the invention

The objective of the present invention is to provide a new compound for preventing and treating liver diseases (such as FLD, hepatic cirrhosis, hepatic fibrosis, etc.) and the pharmaceutical use thereof.

The present invention provides a compound represented by Formula **I-1,** or a pharmaceutically acceptable salt thereof, or a stereoisomer thereof, or a solvate thereof:

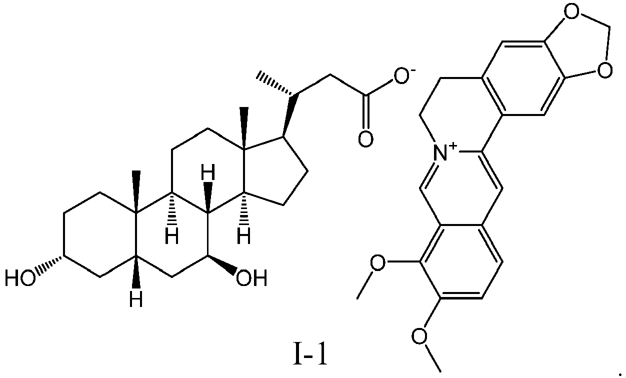

The present invention further provides the use of the compound represented by Formula **I-1,** or a pharmaceutically acceptable salt thereof, or a stereoisomer thereof, or a solvate thereof in the preparation of drugs for the prophylaxis and/or treatment of liver diseases.

Further, the liver diseases include FLD, hepatic fibrosis or hepatic cirrhosis.

Further, said FLD is AFLD or NAFLD.

Further, the drug can reduce ALT, AST and ALP.

The present invention further provides a drug for the prophylaxis and/or treatment of liver diseases, which is a preparation prepared with the compound represented by Formula **I-1,** or a pharmaceutically acceptable salt thereof, or a stereoisomer thereof, or a solvate thereof, as the active ingredient, in combination with pharmaceutically acceptable excipients.

Further, the pharmaceutically acceptable excipients are selected from the group consisting of diluents, bulking agents, colorants, glidants, lubricants, adhesives, stabilizers, suspending agents and buffering agents;
and/or, the dosage form includes tablets, capsules, oral liquid, injections, patches, aerosol, solid preparation, liposome preparation or sustained- and controlled-release preparation;
and/or, each unit of the preparation contains 5 to 2500 mg of the compound represented by Formula **I-1.**

The present invention further provides a preparation method of the compound represented by Formula **I-1,** which comprises the following step: reacting berberine hydrochloride with norursodeoxycholic acid, as the starting materials.

Further, the molar ratio of berberine hydrochloride to norUDCA is 1:(0.8-1.2); the solvent of the reaction is alcohol solvents, and the temperature of the reaction is room temperature.

Further, the molar ratio of berberine hydrochloride to norUDCA is 1: 1; the alcohol solvents are methanol or ethanol.

For the definition of terms used in the present invention, unless otherwise stated, the initial definition provided for the group or term herein is applicable to the group or term in the whole specification; for the terms not specifically defined herein, all terms have the same meaning as commonly understood by one of ordinary skill in the art to which this disclosure belongs, based on the disclosed content and the context.

In the present invention, norursodeoxycholic acid (norUDCA) specifically refers to the loss of one methylene in the side chain of ursodeoxycholic acid, and the total carbon number changes from 24 to 23;

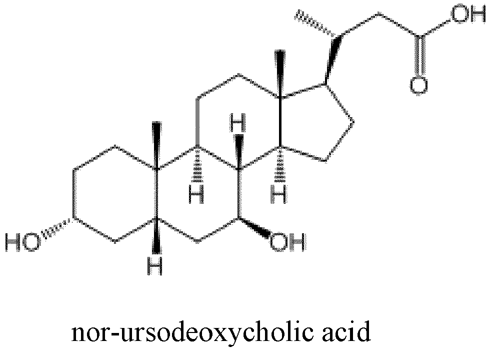

Among the serum biochemical indexes of the present invention, ALT represents glutamic-pyruvic transaminase, AST represents glutamic oxaloacetic transaminase, and ALP represents alkaline phosphatase.

Room temperature refers to 25 ± 2 °C.

As mentioned in the present invention, the solvate refers to that formed by the compound and a solvent, in which the solvent does not exist in free forms, but participates and includes in the crystal to form the solvate. The solvents include (but are not limited to) water, ethanol, methanol, isopropanol, propylene glycol, tetrahydrofuran and dichloromethane.

The experimental results have indicated that the compound of the present invention can not only significantly reduce ALT, AST and ALP, but also have obvious improvement effect on liver pathological damage (such as hepatocyte steatosis). The therapeutic effects of compound **I-1** according to the present invention on FLD, hepatic fibrosis and hepatic cirrhosis are better than the sum of respective therapeutic effects for norUDCA and berberine hydrochloride; the therapeutic effects of compound **I-1** according to the present invention on FLD, hepatic fibrosis and hepatic cirrhosis are significantly better than that of HTD1801. Therefore, the compound of the present invention has broad application prospects in the preparation of drugs for preventing and/or treating FLD, hepatic fibrosis, hepatic cirrhosis and other liver diseases.

Obviously, based on the above content of the present invention, according to the common technical knowledge and the conventional means in the field, other various modifications, alternations or changes can further be made, without department from the above basic technical spirits of the present invention.

By reference to the following specific examples, the above content of the present invention is further illustrated. But it should not be construed that the scope of the above subject matter of the present invention is only limited to the following examples. The techniques realized based on the above content of the present invention are all within the scope of the present invention.

### Description of the Figures

Figure 1. The glutamic oxaloacetic transaminase (AST) index of plasma in the mice of each group in Experimental Example 1.
Figure 2. The glutamic-pyruvic transaminase (ALT) index of plasma in the mice of each group in Experimental Example 1.
Figure 3. The alkaline phosphatase (ALP) index of plasma in the mice of each group in Experimental Example 1.

### Examples

The chemical reagents used in the specific examples of the present invention were purchased from Chengdu Kelong Chemical Reagent Factory.

According to the method in the literature (Journal of Medical Chemistry, 2014: 57: 7687), nor-ursodeoxycholic acid (nor-UDCA) had been prepared, whose NMR spectral data and the same were consistent with those in literature, and the purity was 99.0% by HPLC.

In the following examples, unless otherwise specified, the method used is a conventional method in the art.

### Example 1 Preparation of compound I-1 according to the present invention

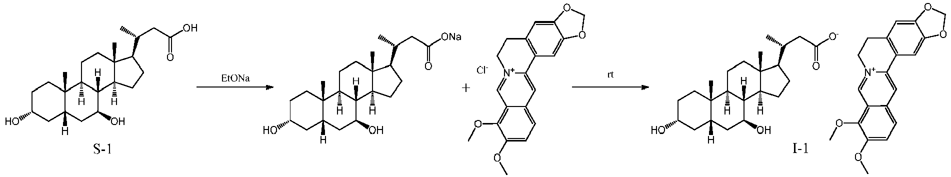

0.79 g of berberine hydrochloride (2.12 mmol) was dissolved in 4 ml of methanol, to which was added sodium sulfate to dry for 60 min, and then the solution was filtered, followed by addition of sodium sulfate. In addition, 0.80 g of nor-UDCA (S-1, 2.11 mmol) was dissolved in 4 ml of ethanol, to which was added 0.15 g of sodium ethoxide (2.20 mmol), and then the mixture was stirred at room temperature for 10 min, and concentrated to dry. The resultant solid was dissolved in 4 ml of methanol, to which was added the solution of berberine hydrochloride in methanol dropwise, and then the mixture was stirred at room temperature for 1 h, and filtered. The filtrate was concentrated to dry. The resultant crude product was dispersed in ethyl acetate and allowed to crystallize, and the collected crystal was dried to obtain compound **I-1** (1.18 g), with a yield of 78.1%.

¹HNMR (DMSO-d₆, 400 MHz): *δ* 9.88 (s, 1H), 8.92 (s, 1H), 8.20 (d, *J* = 9.1 Hz, 1H), 8.00 (d, *J* = 9.1 Hz, 1H), 7.79 (s, 1H), 7.08 (s, 1H), 4.96-4.88 (m, 2H), 4.09 (s, 3H), 4.06 (s, 3H), 3.79 (s, 1H), 3.33-3.23 (m, 2H), 3.23-3.16 (m, 2H), 2.09-2.00 (m, 1H), 1.96-1.88 (m, 1H), 1.84-1.54 (m, 6H), 1.58-1.25 (m, 9H), 1.25-0.91 (m, 7H), 0.91-0.88 (m, 3H), 0.88-0.82 (m, 6H), 0.61 (s, 3H).

### Example 2 Preparation of pharmaceutical tablet composition containing the compound of the present invention

The pharmaceutical tablet composition containing compound **I-1** is composed of compound **I-1** (1 part by weight), lactose (0.1-0.5 parts by weight), hydroxypropylcellulose (0.05-0.08 parts by weight), carboxymethyl starch sodium (0.008-0.014 parts by weight), Povidone K30 (0.01-0.02 parts by weight), and magnesium stearate (0.01-0.05 parts by weight). Tablets were prepared according to the above formula, each containing 50 mg of compound **I-1.**

### Example 3 Preparation of pharmaceutical capsule composition containing the compound of the present invention

For the pharmaceutical capsule composition of compound **I-1,** the raw materials included 100 g of compound **I-1,** 146 g of lactose, 4 g of micronized silica gel, and 2# vacant capsules. The preparation method was:
a. Compound **I-1,** lactose and micronized silica gel were mixed by conventional method, and crushed to obtain the mixture powder;
b. After the mixed powder was filtered through the sieve with 120 meshes, it was filled into a 2# capsule and sealed, and thus a total of 1000 capsules were prepared,
wherein each capsule contained 100 mg of compound **I-1.**

The beneficial effects of the present invention were demonstrated by the following Experimental Examples.

### Experimental example 1 Animal experiment on the prevention and treatment of FLD by the compound of the present invention

### 1. Experimental method

48 male C57BL/6N mice (8-week-old) were chosen, in which 8 mice were randomly divided into the blank group, fed with normal diet, while the other 40 mice were all subjected to establishing models. There was an adaptation period of 6 days before modeling. Then, mice were fed with the mixture of MCD and MCS (TROPHIC Animal Feed High-Tech Co. Ltd, Nantong, Jiangsu, China, TP3005GS, high-fat feed) in a ratio of 1:2, 1:1 and 2:1, and each ratio of diet was given to the mice for two days in the order of 1:2, 1:1 and 2:1. After that, mice were given MCD feed for 28 days (TROPHIC Animal Feed High-Tech Co. Ltd, Nantong, Jiangsu, China, TP3005G, methionine- and choline-deficient diet). On the 28th day, mice were randomly divided into five groups, namely model group, compound **I-1** group, norUDCA group, berberine hydrochloride group, and HTD1801 group, eight mice for each group. After grouping, each treatment group was further fed customized MCD diet containing drugs for 20 days, while the model group was fed the same diet as the previous. All groups were fed the isocaloric diet. After the last administration on the 21st day, the mice were fasted for 16 h (but free access to water) and weighed. Then, blood was taken from the femoral artery and centrifuged to separate the serum, which was stored at low temperature of -30 °C, ready for testing the biochemical indexes of the serum. In addition, the liver was taken, and two pieces of left lobe were taken for each mouse, which were placed in 10% neutral formalin solution, respectively. After treatment, liver histopathological examination was performed.

### 2. Experimental results

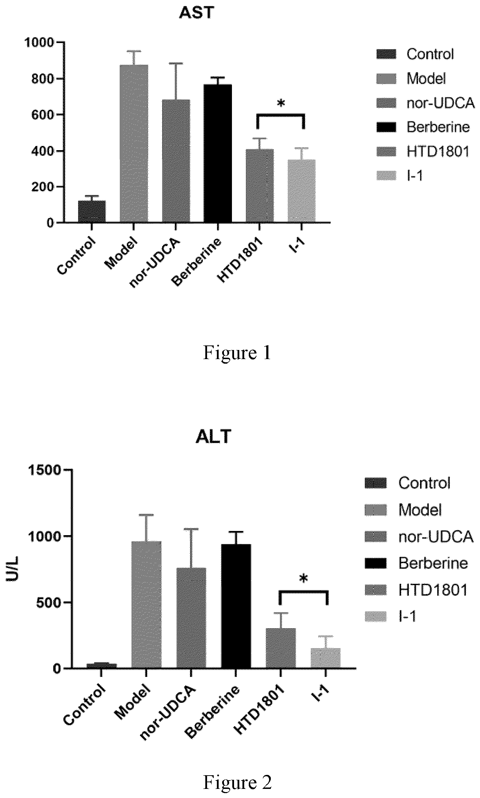

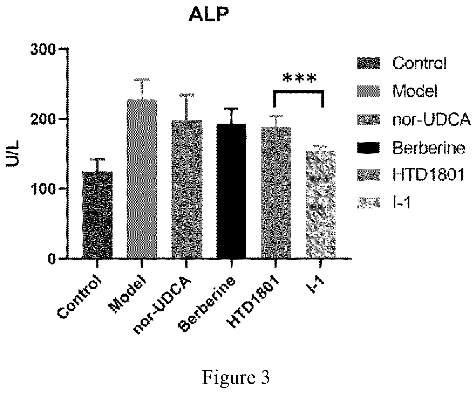

The biochemical indexes of mice in each group are shown in Table 1, Figure 1, Figure 2 and Figure 3:

**Table 1 Serum biochemical indexes of mice in each group**

| Groups | Dosage (mg/kg/d) | AST | ALT | ALP |
|---|---|---|---|---|
| Blank group | N/A | 121.3±7.8 | 35.9±4.7 | 125.7±19.2 |
| Model group | N/A | 874.1±168.8 | 960.3±322.3 | 227.8±41.8 |
| I-1 | 300 | 352.5±49.0^{∗} | 153.3±89.8^{∗} | 154.0±9.7^{∗∗∗} |
| nor-UDCA | 150 | 683.4±253.7 | 761.8±374.2 | 198.3±94.5 |
| Berberine hydrochloride | 150 | 768.4±38.1 | 942.4±64.5 | 193.4±32.9 |
| HTD1801 | 300 | 409.9±51.7 | 303.0±99.1 | 188.8±21.3 |

| | | | | |
|---|---|---|---|---|
| Note: compared with HTD1801, ***P < 0.01, **P < 0.05, *P < 0.5. | | | | |

The results of liver histopathological examination and analysis are shown in Table 2:

**Table 2 Liver histopathological examination of mice in each group**

| Groups | Dosage (mg/kg) | Score | | | |
|---|---|---|---|---|---|
| | | Edema | Steatosis | Inflammation cell infiltration | Necrosis |
| Blank group | N/A | 0 | 0.2 | 0.2 | 0.1 |
| Model group | N/A | 2.2 | 2.9 | 2.1 | 2.5 |
| 1-1 | 300 | 0.5 | 1.0 | 0.9 | 0.7 |
| nor-UDCA | 150 | 1.6 | 2.5 | 2.1 | 2.0 |
| Berberine hydrochloride | 150 | 1.8 | 2.2 | 1.9 | 2.1 |
| HTD1801 | 300 | 0.7 | 1.3 | 1.3 | 0.8 |

The evaluation results of hepatic fat staining are shown in Table 3:

**Table 3 The results of hepatic fat staining for mice in each group**

| Groups | Dosage (mg/kg) | Scores |
|---|---|---|
| Blank group | N/A | 0.1 |
| Model group | N/A | 2.8 |
| I-1 | 300 | 1.0 |
| nor-UDCA | 150 | 1.7 |
| Berberine hydrochloride | 150 | 1.9 |
| HTD1801 | 300 | 1.1 |

The above experiments indicated that compound **I-1** of the present invention could not only significantly reduce ALT, AST and ALP, but also had obvious improvement on the pathological hepatic injury (such as hepatocyte steatosis).

In addition, the therapeutic effect on FLD in compound **I-1** group was significantly better than that in nor-UDCA group, berberine hydrochloride group and HTD1801 group. As shown in Table 1, compared with the model group, the decrease rates of ALT, AST and ALP in nor-UDCA group were 21.82%, 20.67% and 12.95%, respectively, and the decrease rates of ALT, AST and ALP in the berberine hydrochloride group were 12.09%, 1.86% and 15.10%, respectively; however, the decrease rates of ALT, AST and ALP in compound **I-1** group were 59.67%, 84.04% and 32.40%, respectively, higher than the sum of respective decrease rate for nor-UDCA and berberine hydrochloride groups. In addition, compared with HTD1801 group, the effect of lowering ALT, AST and ALP in compound **I-1** group was also significantly improved at the same dosage.

The above experimental results showed that the therapeutic effect of compound **I-1** according to the present invention on FLD was better than the sum of individual treatment effect for nor-UDCA and berberine hydrochloride; the therapeutic effect of compound **I-1** according to the present invention on FLD was significantly better than that of HTD1801.

### Example 2 Investigating on the compound of the present invention for use in the prophylaxis and treatment of thioacetamide-induced hepatic cirrhosis in an animal model

### 1. Experimental methods

60 male Wistar rats (weighing 180-220 g) were divided into 6 groups, with 10 rats in each group, and the rats were provided by Chengdu Dossy Experimental Animal Co., Ltd. In the experiment, the compound of the present invention and the positive control drug nor-UDCA were first ground in a proper amount of Tween, and then diluted to the pre-determined concentration with 0.5% carboxymethyl cellulose (CMC) sodium.

The rat liver fibrosis model was established by inducing with thioacetamide. Rats were intraperitoneally injected with thioacetamide at a dose of 200 mg/kg, three times a week, for 12 weeks. Four weeks after induction with thioacetamide, the compound of the present invention was orally administered to the test groups, while the blank group and the model group were given the same amount of CMC suspension. Among them, every morning, rats in each test group were given 160 mg/kg of compound **I-1,** 80 mg/kg of nor-UDCA, 80 mg/kg of berberine hydrochloride, and 160 mg/kg of positive control HTD1801. After experiment, all rats were euthanized, and their livers were taken out.

The liver tissue was fixed with 10% formaldehyde solution and embedded in paraffin. The fixed liver tissue was sectioned with a thickness of about 5 mm, and then stained with Hematoxylin-Eosin (HE) and Sirius Red. Sirius Red staining was used for morphological analysis and to determine the percentage of liver tissue affected by its fibrosis. The image of the section was collected by using the microscopic imaging system. In order to make the results more accurate, 15 positive visual fields of Sirius Red staining were randomly selected from each slice to calculate the proportion of liver fibrosis area, which was used in statistical analysis.

### 2. Experimental results

The experimental results are shown in Table 4:

**Table 4 Hepatic fibrosis area of rats in each group**

| Groups | Dosage (mg/kg) | Hepatic fibrosis area (%) |
|---|---|---|
| Blank group | N/A | 0.0 ±0.0 |
| Model group | N/A | 17.80±4.15 |
| I-1 | 160 | 8.72±1.06^{∗} |
| nor-UDCA | 80 | 16.79±5.48 |
| Berberine hydrochloride | 80 | 15.14±3.67 |
| HTD1801 | 160 | 9.39±1.45 |

| | | |
|---|---|---|
| Note: compared with HTD1801, **P < 0.05, *P < 0.5. | | |

The results indicated that the compound of the present invention could significantly inhibit hepatic fibrosis induced by thioacetamide. In addition, as shown in Table 4, compared with the rat cirrhosis model group, the decrease rate of fibrosis area in nor-UDCA group was 6.18%, the decrease rate of fibrosis area in berberine hydrochloride group was 15.17%, and the decrease rate of fibrosis area in compound **I-1** group was 50.90%, which was better than the sum of respective decrease rate for nor-UDCA group and berberine hydrochloride group. Compared with HTD1801 group, the fibrotic area of compound **I-1** group at the same dosage was also significantly reduced.

The above experimental results suggested that the anti-liver fibrosis effect of compound **I-1** according to the present invention was better than the sum of respective therapeutic effect for nor-UDCA and berberine hydrochloride; the anti-fibrosis effect of compound **I-1** of the present invention was significantly better than that of HTD 1801.

In sum, the present invention provides an ionic salt compound of nor-UDCA. The experimental results indicated that the compound of the present invention could not only significantly reduce ALT, AST and ALP, but also significantly improve the pathological injuries on livers (such as hepatocyte steatosis). The therapeutic effects of compound **I-1** of the present invention on FLD, hepatic fibrosis and cirrhosis were better than the sum of respective effect for nor-UDCA and berberine hydrochloride; and the therapeutic effects of compound **I-1** of the present invention on FLD, hepatic fibrosis and cirrhosis were significantly better than that of HTD1801. Therefore, the compound of the present invention has broad application prospects in the preparation of drugs for preventing and/or treating FLD, hepatic fibrosis, hepatic cirrhosis and other hepatic diseases.

## Claims

1. A compound represented by Formula **I-1,** or a pharmaceutically acceptable salt thereof, or a stereoisomer thereof, or a solvate thereof:

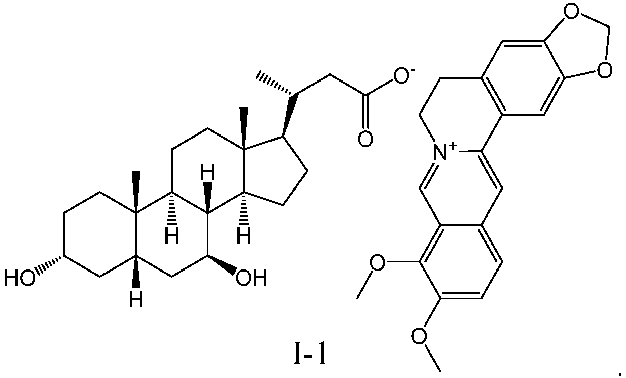

2. Use of the compound according to claim 1, or a pharmaceutically acceptable salt thereof, or a stereoisomer thereof, or a solvate thereof in the preparation of drugs for the prophylaxis and/or treatment of hepatic diseases.

3. The use according to claim 2, **characterized in that** the hepatic diseases include fatty liver disease (FLD), hepatic fibrosis or hepatic cirrhosis.

4. The use according to claim 3, **characterized in that** the fatty liver disease is alcoholic fatty liver disease or nonalcoholic fatty liver disease.

5. The use according to any one of claims 2 to 4, **characterized in that** the drug can reduce ALT, AST and ALP.

6. A drug for the prophylaxis and/or treatment of hepatic diseases, **characterized in that** the drug is a preparation prepared with the compound according to claim 1, or a pharmaceutically acceptable salt thereof, or a stereoisomer thereof, or a solvate thereof, as the active ingredient, in combination with pharmaceutically acceptable excipients.

7. The drug according to claim 6, **characterized in that**
the pharmaceutically acceptable excipients are selected from the group consisting of diluents, bulking agents, colorants, glidants, lubricants, adhesives, stabilizers, suspending agents and buffering agents;
and/or,
the dosage form includes tablets, capsules, oral liquid, injections, patches, aerosol, solid preparation, liposome preparation or sustained- and controlled-release preparation;
and/or,
each unit of the preparation contains 5 to 2500 mg of the compound according to claim 1.

8. A preparation method for the compound according to claim 1, **characterized in that** the method comprises the following step: reacting berberine hydrochloride and norursodeoxycholic acid, as the starting materials.

9. The method according to claim 8, **characterized in that** the molar ratio of berberine hydrochloride to norursodeoxycholic acid is 1:(0.8-1.2); the solvent of the reaction is an alcohol solvent, and the temperature of the reaction is room temperature.

10. The method according to claim 9, **characterized in that** the molar ratio of berberine hydrochloride to norursodeoxycholic acid is 1:1; the alcohol solvent is methanol or ethanol.
